# EUROPEAN PATENT APPLICATION

(11) **EP 3 686 283 A1**
(43) Date of publication of application: **29.07.2020**
(21) Application number: 20152852.8
(22) Date of filing: 21.01.2020
(51) Int. Cl.: C12P 23/00, C12N 1/12, C12N 13/00, A01G 33/00

(54) **A METHOD FOR ENHANCEMENT OF PRODUCTIVITY IN MICROALGAE**

(30) Priority: 22.01.2019 IN 201921002604
(71) Applicant: Reliance Industries Limited, Mumbai 400 021 Maharashtra (IN)
(72) Inventor: Talukdar, Jayanta, 781021 Guwahati (IN); Banerjee, Arun, 410210 Kharghar (IN); Paul, Kenny, 678001 Palakkad (IN); Laxman Nagle, Vinod, 401105 Bhayander (East) Thane (IN); Dasgupta, Santanu, 400076 Mumbai (IN)
(74) Representative: Nederlandsch Octrooibureau

(57) **Abstract**

The instant disclosure relates to the field of algal cultivation and production of high value biochemical products thereof. Particularly, the present disclosure relates to a cultivation method comprising the application of red light/far-red light in life cycle management of green microalgae, particularly *Haematococcus*, including induction of intense vegetative growth, and enhancement of productivity. The present method is simple, commercially scalable and cost-effective, achieves enhanced productivity, and requires shorter time duration, amongst other advantages.

## Description

### TECHNICAL FIELD

. The instant disclosure relates to the field of algal cultivation and production of high value biochemical products thereof. The present disclosure relates to a cultivation method comprising the application of red light and/or far-red light in life cycle management of green microalgae, including induction of intense vegetative growth and enhancement in productivity.

### BACKGROUND

. Carotenoids such as astaxanthin (C₄₀H₅₂O₄, 3,3'-dihydroxy-β,β'-carotene-4,4'-dione) is a xanthophyll keto-carotenoid, best known for giving the pinkish-red coloration to salmons, trouts, shrimps, lobsters and crayfishes, along with its central role for their immune-system and positive fertility impacts. While the current major commercially available source of astaxanthin is the synthetic form of this pigment with a market value of around US$ 3000 per kilogram, but said synthetic form may contain astaxanthin compounds having unnatural configuration. Besides, the chemically synthesized astaxanthin is from petrochemicals, whereas, naturally astaxanthin is produced by microalgae, yeast and bacteria. Natural astaxanthin with a current market value around US$ 10000 per kilogram is proven to be more effective and generally considered to be safe for human consumption. From human nutritional point of view, astaxanthin is considered as the most powerful antioxidant in nature serving the role of a highly efficient scavenger of free radicals. Multiple clinical studies have proven that astaxanthin protects the skin against UV-induced photo-oxidation and is used as anti-aging, anti-tumor, anti-diabetic, prevention and treatment of neural damage interrelated with age-related macular degeneration, Alzheimer's and Parkinson's diseases. Further, astaxanthin is considered as a natural superfood destined to enhance athletic performance by increasing stamina and reducing the time of muscle recovery. Based on its wide scale applications as nutraceuticals, cosmeceuticals and pharmaceuticals ingredient, astaxanthin has a very high commercial potential.

. The green microalga *Haematococcus* is currently the richest natural source of astaxanthin, which has been utilized for commercial scale production of natural astaxanthin. However, comparatively slow growth rate, low biomass productivity and subsequently extended durations of production cycle are the principal process bottlenecks in cost effective commercial/large-scale scale cultivation. *Haematococcus* sp., particularly *Haematococcus pluvialis* consists of a unique and highly complex life cycle with distinct morphological stages: (1) Vegetative green flagellates, (2) Intermediate palmelloids, and (3) Haematocysts (aplanospores), of which only the cyst form (i.e. aplanospores) accumulate massive amount of astaxanthin. Conventional approach of commercial astaxanthin production is mostly governed by morphogenetic changes of the life cycle stages either naturally or by induced growth conditions, which itself lasts at least 15 to 30 days. In such commercial cultivation, a two-stage approach is employed, involving biomass growth followed by intermediate harvesting to remove excess nutrients followed by exposure to stress conditions, mostly nitrogen deficient conditions, addition of induction nutrients (micronutrients, vitamins, or increasing salinity, etc.) and chemicals (hydrogen peroxide, hypochlorite, jasmonic acid, etc.) followed by exposure to high light stress for astaxanthin accumulations. Although implication of nutrient limitation is commonly applied, but said approach involves a huge operational cost and complexity to preserve highly sensitive flagellate cells, failure of which results in considerable loss of biomass along with astaxanthin productivities, and hence a reduction of overall yield.

. Hence, a method to control the life cycle stages and thereby bypassing the normal life cycle with a modified short cycle may enhance the productivities as well as improve carotenoid, especially astaxanthin production, and may also substantially reduce the total operational cost. The present disclosure therefore is related to the said need of developing a simple, cost-effective and more efficient method for enhancing biomass growth and subsequent carotenoid yields.

### SUMMARY

. The present disclosure relates to a method of concomitant enhancement of cell biomass and carotenoid production in green-microalgae, particularly *Haematococcus* sp.

. In an embodiment, the present disclosure relates to a method of concomitant enhancement of cell biomass and carotenoid production in *Haematococcus* sp. comprising applications of monochromatic light selected from far-red light, red light, or a combination of the far-red light and the red light.

. In yet another embodiment of the present disclosure, the carotenoid is selected from a group comprising astaxanthin, lutein, β-carotene, canthaxanthin, lycopene, violaxanthin, zeaxanthin and combinations thereof, preferably astaxanthin.

. In still another embodiment of the present disclosure, the *Haematococcus* sp. is *Haematococcus pluvialis.*

. In a preferred embodiment, the present method comprises exposing *Haematococcus* cells to a combination of monochromatic far-red light and monochromatic red light at a wavelength range between 630 nm to 800 nm, preferably about 640 nm to 760 nm for concomitant enhancement of cell biomass and carotenoid production.

. In an exemplary embodiment, the present method for concomitant enhancement of cell biomass and carotenoid production in *Haematococcus* sp., comprises steps of:
a. exposing *Haematococcus* cells to white light, or monochromatic far-red light, or a combination of the white light and the monochromatic far-red light, to obtain cell biomass;
b. exposing the cell biomass to monochromatic far-red light, or monochromatic red light or, preferably a combination of the monochromatic far-red light and the monochromatic red light, for concomitant enhancement of cell biomass and carotenoid production.

. In another embodiment, the present method is completed in a time-period ranging from about 4 days to 6 days.

### BRIEF DESCRIPTION OF THE ACCOMPANYING FIGURES

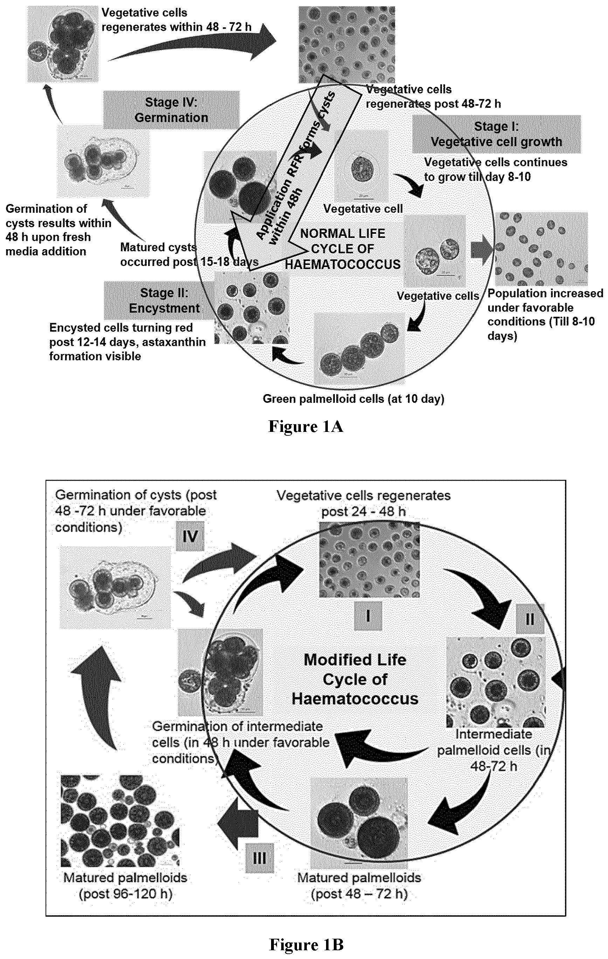

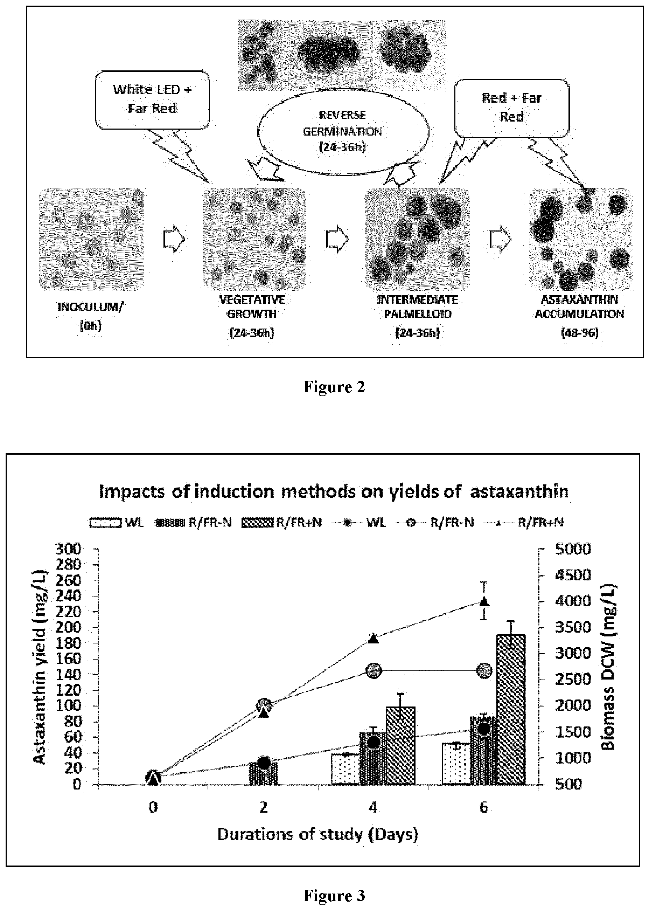

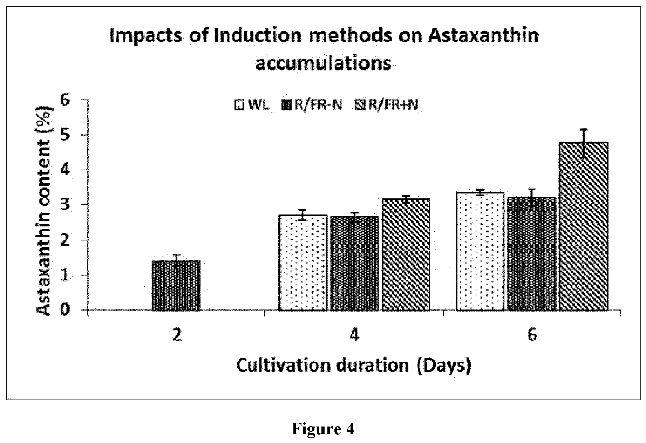

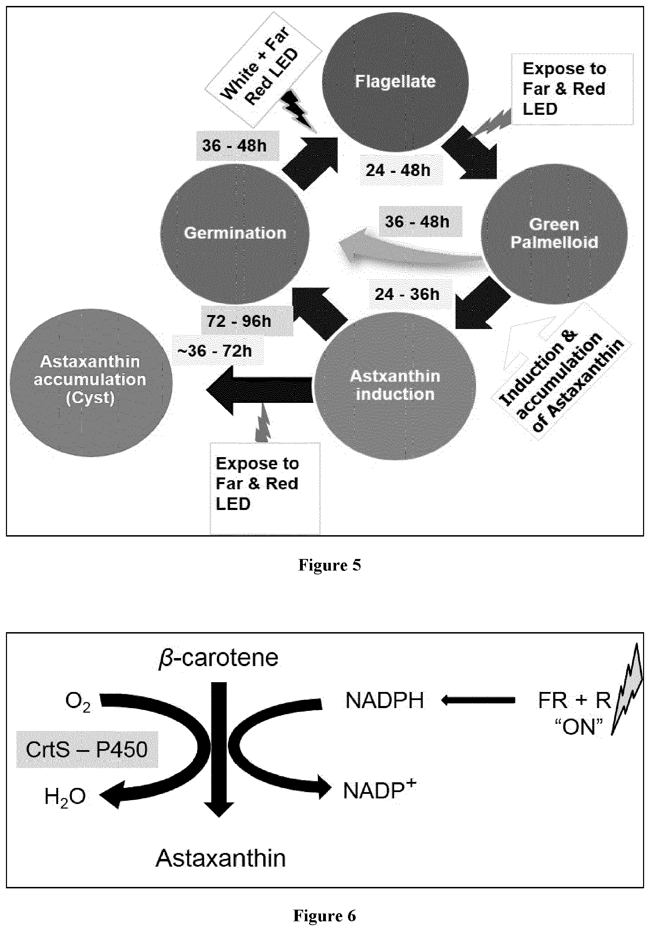

. In order that the disclosure may be readily understood and put into practical effect, reference will now be made to exemplary embodiments as illustrated with reference to the accompanying figures. The figures together with detailed description below, are incorporated in and form part of the specification, and serve to further illustrate the embodiments and explain various principles and advantages, where:
**.** **Figure 1**(A-B) depicts the control and management of life cycle stages of *Haematococcus.* The present method involving application of monochromatic Red and/or Far-red Light, preferably a combination of Red and Far-red Light modifies the normal life cycle (represented under Figure 1A) by precise control over the morphogenetic changes of *Haematococcus* which enables rapid production/growth of vegetative cells and shortens the period of astaxanthin accumulating cysts formation compared to its normal life cycle (modified life cycle shown under Figure 1B).
**.** **Figure 2** depicts rapid morphogenetic changes of life cycle stages by application of present method involving far-red exposure. Intensive vegetative growth of flagellates and subsequent morphogenetic changes to intermediate palmelloids and astaxanthin accumulating cysts cells were triggered with the supplementation of far-red light either with white light and red light, respectively. Intense vegetative growth and astaxanthin accumulating cysts were triggered at any point of time regardless of periods of its normal life cycle and independent to nutrient status.
**.** **Figure 3** depicts expedited astaxanthin production cycle through one-phase red/far-red cultivation method of the present disclosure. Concomitant enhancement of biomass growth and rapid astaxanthin production was observed in *Haematococcus* cells with supplementation of red and far-red light (R/FR) under defined nutrient sufficient conditions. Biomass productivities and astaxanthin content enhanced rapidly under R/FR exposure. Astaxanthin production cycle shortened up to about 6-7 days with a yield of astaxanthin nearly about 3-4 times higher than conventional method employing white light.
**.** **Figure 4** depicts the impacts of induction methods on astaxanthin accumulation. Red and Far-red exposure results in rapid induction within 72 hours with improved astaxanthin accumulation over 5% in *Haematococcus* under nutrient sufficient conditions.
**.** **Figure 5** provides a schematic outline of transition period and controlling of various morphogenetic stages of *Haematococcus* cells based on the present method. The present method involves advanced cultivation strategy based on R/FR applications promoting concurrent biomass and rapid astaxanthin production.
**.** **Figure 6** depicts the probable working model for far-red and red light mediated astaxanthin induction of the method of present disclosure.

### DESCRIPTION

**.** To address the limitations as stated in the background, the present disclosure provides a method for concomitant enhancement of biomass growth along with carotenoid accumulation in green microalgae.

**.** In an embodiment, the present disclosure relates to a method for concomitant enhancement of biomass growth along with carotenoid accumulation in *Haematococcus* sp.

**.** In an exemplary embodiment, the present disclosure provides a cultivation method involving application of monochromatic far-red light and monochromatic red light in the life cycle management of green microalgae, preferably *Haematococcus* sp., in inducing intense vegetative growth, and concurrent enhancement of cell biomass and carotenoid accumulations.

**.** As used herein, the feature 'concomitant enhancement of cell biomass and carotenoid production' refers to biomass associated carotenoid production, wherein the method of present disclosure simultaneously results in enhanced biomass and carotenoid production.

. The present invention relates to a method for concomitant enhancement of cell biomass and carotenoid production in in microalgal cells, said method comprising exposing the microalgal cells to monochromatic light selected from red light, far-red light, or a combination thereof.

. The present disclosure particularly relates to a method for concomitant enhancement of cell biomass and carotenoid production in *Haematococcus* sp., said method comprising exposing the *Haematococcus* cells to monochromatic light selected from far-red light, red light, or a combination thereof.

. In an exemplary embodiment of the present disclosure, the *Haematococcus* sp. is *Haematococcus pluvialis.*

. In another embodiment of the present disclosure, the *Haematococcus* cells are exposed to monochromatic far-red light at a wavelength ranging from about 700 nm to 800 nm, preferably about 730-760 nm.

. In yet another embodiment of the present disclosure, the *Haematococcus* cells are exposed to monochromatic red light at a wavelength ranging from about 630 nm to 700 nm, preferably about 640-680 nm.

. In still another embodiment of the present disclosure, the *Haematococcus* cells are exposed to a combination of monochromatic far-red light and monochromatic red light at a wavelength ranging from about 630 nm to 800 nm, preferably about 640 nm to 760 nm.

. In still another embodiment of the present disclosure, the *Haematococcus* cells are exposed to monochromatic light selected from far-red light, red light or a combination thereof, for a time-period ranging from about 12 hours to 192 hours, preferably about from 48 hours to 144 hours.

. In still another embodiment of the disclosure, the present method enhances production of carotenoid selected from a group comprising astaxanthin, lutein, β-carotene, canthaxanthin, lycopene, violaxanthin, zeaxanthin and combinations thereof, preferably astaxanthin.

. In still another embodiment of the disclosure, the microalgae is *Haematococcus* sp., preferably *Haematococcus pluvialis,* and the carotenoid selected from a group comprising astaxanthin, lutein, β-carotene, canthaxanthin, lycopene, violaxanthin, zeaxanthin and combinations thereof, preferably astaxanthin.

. In still another embodiment of the disclosure, the carotenoid is astaxanthin.

**.** In still another embodiment of the disclosure, the present method for concomitant enhancement of cell biomass and carotenoid production in *Haematococcus* sp., comprises steps of:
a. exposing *Haematococcus* cells to white light, or monochromatic far-red light, or a combination of the white light and the monochromatic far-red light, to obtain starting seed cell biomass or inoculum;
b. exposing the cell biomass of step a) to monochromatic far-red light, or monochromatic red light, or a combination of the monochromatic far-red light and the monochromatic red light, for concomitant enhancement of cell biomass and carotenoid production.

. In still another embodiment of the present disclosure, the exposure to white light in step (a) of the above described method is at a wavelength ranging from about 340 nm to 850 nm, and wherein the exposure to monochromatic far-red light in step (a) is at a wavelength ranging from about 700 nm to 800 nm, preferably about 730-760 nm.

. In still another embodiment of the present disclosure, the step (a) of the above described method comprises inoculating the *Haematococcus* cells in a cell culture media and exposing under suitable conditions for a time-period ranging from about 48 hours to 72 hours to achieve optimal vegetative cell growth for starting seed cell biomass or inoculum production, wherein said suitable conditions comprise growing the cells under monochromatic far-red light at a wavelength between 700 nm to 800 nm or a 1:1 combination of cool white light and monochromatic far-red light at a wavelength between 340 nm to 850 nm, a light intensity in the range of about 80 to 200 *µ*mol photons.m⁻²s⁻¹, preferably in the range of about 100-160 *µ*mol photons.m⁻²s⁻¹; a photoperiod of about 12:12 or 14:10 or 16:8 or 18:6 or 24:0 hours (Light:Dark) cycle, preferably 18:6 hours, a temperature in the range of about 25°C to 28°C, a pH in the range of about 7.2 to 7.5, and a continuous supply of air containing about 2% to 5% CO₂.

. In still another embodiment of the present disclosure, the step (b) of the above described method comprises: (i) exposing the *Haematococcus* cells grown according to the step (a) directly under continuous combination of monochromatic far-red light and monochromatic red light at 1:1, 1:2, 2:1, 3:1 or 1:3 ratios, preferably at 2:1 ratio, and a wavelength between 630 nm to 800 nm, preferably 640 nm to 760 nm, for a time-period ranging from about 12 hours to 192 hours, preferably about 48 hours to 144 hours, a light intensity in the range of about 50 to 300 *µ*mol photons.m⁻²s⁻¹, a photoperiod of 12:12 or 14:10 or 16:8 or 18:6 or 24:0 hours (Light:Dark) cycle, preferably 24:0 hours, a temperature in the range of about 25°C to 28°C, a pH in the range of about 7.2 to 7.5, and a continuous supply of air containing about 2% to 5% CO₂, to achieve concomitant enhancement of cell biomass and astaxanthin production; or (ii) inoculating the *Haematococcus* cells grown according to the step (a) in a cell culture media, and exposing under suitable conditions for a time-period ranging from about 12 hours to 192 hours to achieve concomitant enhancement of cell biomass and carotenoid production, wherein said suitable conditions comprise growing the cells under the combination of monochromatic far-red light and monochromatic red light at 1:1, 1:2, 2:1, 3:1 or 1:3 ratios, preferably at about 2:1 ratio, a wavelength between 630 nm to 800 nm, preferably 640 nm to 760 nm, a light intensity in the range of about 50 to 300 *µ*mol photons.m⁻²s⁻¹, a photoperiod of 12:12 or 14:10 or 16:8 or 18:6 or 24:0 hours (Light:Dark) cycle, preferably 24:0 hours, a temperature in the range of about 25°C to 28°C, a pH in the range of about 7.2 to 7.5, and a continuous supply of air containing about 2% to 5% CO₂.

. In still another embodiment of the present disclosure, the *Haematococcus* cells cultured in the step (a) of the above described method are pre-encysted vegetative cells.

**.** In still another embodiment of the disclosure, the present method comprises concomitant enhancement of cell biomass and carotenoid production in *Haematococcus pluvialis,* comprising steps of:
a. exposing *Haematococcus pluvialis* cells to white light, or monochromatic far-red light, or a combination thereof, preferably the combination of white light and the monochromatic far-red light, to obtain starting seed cell biomass or inoculum; and
b. exposing the cell biomass of step a) to monochromatic far-red light, or monochromatic red light or a combination thereof, preferably the combination of monochromatic far-red light and the monochromatic red light, for concomitant enhancement of cell biomass and carotenoid production.

. In still another embodiment of the present disclosure, the above described method comprises concomitant enhancement of cell biomass and astaxanthin production in *Haematococcus pluvialis.*

**.** In an exemplary embodiment of the disclosure, the present method comprises concomitant enhancement of cell biomass and astaxanthin production in *Haematococcus pluvialis,* comprising steps of:
a. inoculating the *Haematococcus pluvialis* cells in a cell culture media and growing under suitable conditions for a time-period ranging from about 48 hours to 72 hours to achieve optimal vegetative cell growth to obtain starting seed cell biomass or inoculum, wherein said suitable conditions comprise growing the cells under 1:1 combination of cool white light and monochromatic far-red light at a wavelength between 340 nm to 850 nm, a light intensity in the range of about 80-200 *µ*mol photons.m⁻²s⁻¹, preferably about 100 to 160 *µ*mol photons.m⁻²s⁻¹, a photoperiod of 12:12 or 14:10 or 16:8 or 18:6 or 24:0 hours (Light:Dark) cycle, preferably 18:6 hours, a temperature in the range of about 25°C to 28°C, a pH in the range of about 7.2 to 7.5, and a continuous supply of air containing about 2% to 5% CO₂; and
b. inoculating the *Haematococcus pluvialis* cells grown according to the step (a) in a cell culture media, and growing under suitable conditions for a time-period ranging from about 12 hours to 192 hours, preferably about 48 hours to 144 hours to achieve concomitant enhancement of cell biomass and astaxanthin production, wherein said suitable conditions comprise growing the cells under continuous exposure of the combination of monochromatic far-red light and red light preferably at about 2:1 ratio, a wavelength between 630 nm to 800 nm, preferably between 640 nm to 760 nm, a light intensity in the range of about 50 to 300 *µ*mol photons.m⁻²s⁻¹, a photoperiod of about 12:12 or 14:10 or 16:8 or 18:6 or 24:0 hours (Light:Dark) cycle, preferably 24:0 hours, a temperature in the range of about 25°C to 28°C, a pH in the range of about 7.2 to 7.5, and a continuous supply of air containing about 2% to 5% CO₂.

**.** In another exemplary embodiment of the disclosure, the present method comprises concomitant enhancement of cell biomass and astaxanthin production in *Haematococcus pluvialis,* comprising steps of:
a. inoculating the *Haematococcus pluvialis* cells in a cell culture media and growing under suitable conditions for a time-period ranging from about 48 hours to 72 hours to achieve optimal vegetative cell growth to obtain starting seed cell biomass or inoculum, wherein said suitable conditions comprise growing the cells under 1:1 combination of cool white light and monochromatic far-red light at a wavelength between 340 nm to 850 nm, a light intensity in the range of about 80-200 *µ*mol photons.m⁻²s⁻¹, preferably about 100 to 160 *µ*mol photons.m⁻²s⁻¹, a photoperiod of 12:12 or 14:10 or 16:8 or 18:6 or 24:0 hours (Light:Dark) cycle, preferably 18:6 hours, a temperature in the range of about 25°C to 28°C, a pH in the range of about 7.2 to 7.5, and a continuous supply of air containing about 2% to 5% CO₂; and
b. exposing the *Haematococcus* cells grown according to the step (a) directly under continuous combination of monochromatic far-red light and monochromatic red light at 1:1, 1:2, 2:1, 3:1 or 1:3 ratios, preferably at 2:1 ratio, and a wavelength between 630 nm to 800 nm, preferably 640 nm to 760 nm for a time-period ranging from about 12 hours to 192 hours, preferably about 48 hours to 144 hours, a light intensity in the range of about 50 to 300 *µ*mol photons.m⁻²s⁻¹, a photoperiod of 12:12 or 14:10 or 16:8 or 18:6 or 24:0 hours (Light:Dark) cycle, preferably 24:0 hours, a temperature in the range of about 25°C to 28°C, a pH in the range of about 7.2 to 7.5, and a continuous supply of air containing about 2% to 5% CO₂; to achieve concomitant enhancement of cell biomass and astaxanthin production.

**.** In still another embodiment of the disclosure, the present method is completed in a time-period ranging from about 2 days to 8 days, preferably in the range from 4 days to 6 days.

**.** In still another embodiment of the present method, the average biomass growth rate ranges from about 0.5 to 0.7 gram/litre/day (gL⁻¹d⁻¹), and the astaxanthin yield ranges from about 150 to 270 mgL⁻¹.

**.** In still another embodiment of the disclosure, the present method is performed in a cultivation apparatus or a photobioreactor (PBR) system selected from a group comprising Erlenmeyer flask/Conical flask, vertical photobioreactor, tubular photobioreactor, flat panel photobioreactor, and combinations thereof.

**.** In still another embodiment of the disclosure, the present method is completed in a time-period ranging from about 2 days to 8 days, preferably in the range from 4 days to 6 days; the method is performed in a cultivation apparatus or a photobioreactor (PBR) system selected from a group comprising Erlenmeyer flask/Conical flask, vertical photobioreactor, tubular photobioreactor, flat panel photobioreactor and combinations thereof; and the average biomass growth rate ranges from about 0.5 to 0.7 gram/litre/day (gL⁻¹d⁻¹), and the astaxanthin yield ranges from about 150 to 270 mgL⁻¹.

**.** The present disclosure in particular discloses a unique approach for life cycle management of phototrophic and/or mixotrophic microalgae, especially *Haematococcus* sp. *via.* modulating highly light sensitive photoreceptors to possibly control the cellular and physiological activities thereby allowing concomitant enhancement of biomass and carotenoid (more particularly, astaxanthin) production. Accordingly, the present disclosure provides a procedure for producing xanthophyll pigments (particularly, astaxanthin) from said microalgae, which includes: (1) inoculating the microalgae, preferably pre-encysted vegetative cells in optimal nutrient and growth conditions, exposing to a combination of white light (340 to 850 nm) and monochromatic far-red light (700 to 800 nm) for intense vegetative growth; followed by (2) exposing the biomass to a combination of monochromatic red light and far-red light (630 to 800 nm) with one third of the nutrient content of the optimal nutrient contents for biomass growth, morphogenetic changes and induction of astaxanthin accumulating encysted cells.

**.** The method of the present disclosure aims to follow a strategically modified form of life cycle of the organism (more particularly *Haematococcus* sp.) by: (A) triggering the transformation of vegetative flagellated cells to pre-encysted intermediate palmelloid cells within 24 to 36 hours in nutrient sufficient conditions at any point of time of its life cycle, (B) which can be either partly reverse back to introducing to the inoculum development process that undergo multiple germination under optimal conditions, and (C) induction of improved carotenoid (particularly, astaxanthin) accumulation within 48 hours post encysted cells without removal of the growing nutrient medium, thereby ensuring that the chances of biomass loss is negligible.

**.** The present disclosure particularly provides a method for efficient production of secondary carotenoid rich cell biomass. More particularly, the present method discloses a single stage rapid method for achievement of concurrent biomass growth and astaxanthin accumulations under nutrient sufficient conditions. The overall astaxanthin production cycle, including biomass growth and astaxanthin accumulations last only for about 6 to 7 days. Overall biomass productivity by employing the present method is improved (up to 2 to 3 fold), with nearly 2 fold increase in astaxanthin content (up to 5.8%) and over 3 fold increase in astaxanthin yield (up to 270 mgL⁻¹). Compared to conventional methods, neither intermediate or primary harvesting for nutrient removal nor suspending to a defined encysting medium is necessary in the present method. Thus, the present disclosure provides a novel one stage cultivation method comprising enhancement of both biomass production as well as astaxanthin accumulations concurrently under appropriate nutrient sufficient conditions that substantially reduce the total durations of astaxanthin production cycle to around 6 to 7 days compared to conventional method which require about 15 to 30 days, thereby resulting in significant reduction in process time, cost and chances of contaminations.

**.** In an embodiment, the present disclosure provides a method to control and manage the life cycle stages of *Haematococcus* for concomitant production of cell biomass and carotenoid-containing cells, especially the keto-carotenoid astaxanthin by modulating qualities of light spectrum, in particular, by employing a proportionate mixture of white light and monochromatic far-red light of wavelength between 340 to 850 nm, and, a mixture of monochromatic far-red light and monochromatic red light of wavelength between 630 to 800 nm. The invention discloses methods for intense vegetative growth under cultivation of non-astaxanthin accumulating phase of photoautotrophic microalgae belonging to the class Chlorophyceae, more particularly, *Haematococcus* sp. The invention further comprises methods of generating intermediate cells and/or expedite carotenoid accumulations simultaneously with biomass growth, resulting in high yields of carotenoid rich biomass, in particular astaxanthin, by employment of strategic combinations of monochromatic far-red and monochromatic red (630 to 800 nm) lights in the shortest plausible time compared to the conventional methods of cultivating microalgae species, preferably *Haematococcus pluvialis.* Especially, the present method controls the life cycle stages of *Haematococcus* sp. and thereby bypassing the normal life cycle with a modified short cycle enhances the productivities as well as improved production yields (Figure 5).

. In an exemplary embodiment, the present disclosure relates to a method involving the employment of monochromatic far-red light, either alone or supplemented with either monochromatic red light or white light to manage/control the transition over flagellate, palmelloid and cysts (red) formation in *Haematococcus* sp., which is highly advantageous in the process point of view for faster inoculum development, ease of harvesting and reduced durations of production cycle.

. The employment of monochromatic far-red light or a combination of monochromatic far-red light and monochromatic red light in *Haematococcus* cultivation for astaxanthin production was unexplored. Without wishing to be bound by any theory, the present inventors hypothesize that the combination of far-red and red light activates beta ketolase hydrolase (CrtS) enzyme activity through NADPH and triggering retrograde signaling pathway from chloroplast to nucleus (Figure 6). The ratio of red/far-red is also related to the circadian rhythm and the transition between the physiologically inactive form absorbing red and a physiologically active form absorbing far-red light. As a result, providing these two light forms in the diurnal cycle can cause transition from one form to another in the process of photo-conversion. Increase in carotenoid accumulation, more particularly astaxanthin accumulation, may thereby related to the same mechanism. The key attributes of monochromatic far-red light exposure could be excitation of photosystem I and thereby increasing the NADPH pool, which helps the NADPH dependent CrtS enzyme activity. This enzyme has both beta-hydroxylase and ketolase activity and is required for astaxanthin production. Another important role of far-red light could be modulating retrograde signaling between chloroplast and nucleus, and thereby impacting cell phase and/or morphology. Further, the color of the light spectrum has a decisive effect on photo-morphogenesis.

. Without wishing to be bound by any theory, the present inventors additionally hypothesize that the combination of far-red light and red light effects photosystem I [PS-I] and photosystem II [PS-II] stoichiometry, i.e. PS-I/PS-II ratio and thereby modulates carotenoid gene expression in the carotenoid biosynthetic pathway which helps in triggering astaxanthin production in *Haematococcus.*

. In another embodiment, a new single-phase cultivation process for large growth of *Haematococcus* cells for large-scale commercial astaxanthin production is provided. In particular, a unique process for the large-scale production of astaxanthin rich *Haematococcus* biomass is provided, which utilizes the aforesaid rapid one-phase cultivation process by strategically employing monochromatic far-red light, either alone or supplemented with either monochromatic red light or white light.

. In an exemplary embodiment, the present disclosure provides a procedure for producing xanthophyll pigments from photosynthetic microalgae, more preferably *Haematococcus* sp., which includes: (1) inoculating the microalgae, preferably pre-encysted vegetative cells in optimal nutrient and growth conditions, and exposing to white light (340-850 nm) and/or supplemented with monochromatic far-red light (700-800 nm) for intense vegetative growth supporting maintenance and continuous generation of starting seed culture (inoculum), which ensures the continuous supply of seed culture at desired volume of cell biomass and nutrient contents, preferably in between 0.55 - 0.75 gL⁻¹ cell biomass and 1/3^{rd} of optimal nutrients, preferably nitrogen (N) content in between 30-40 mgL^{-l} respectively; followed by (2) exposing the cell biomass to monochromatic far-red light, monochromatic red light, or a combination thereof, preferably the combination of monochromatic far-red light and monochromatic red light (630-800 nm) for biomass growth and subsequent morphogenetic changes and rapid induction of astaxanthin accumulating encysted cells.

. In another exemplary embodiment, the present disclosure provides a method for cultivating *Haematococcus* cells in a single phase for large-scale production of astaxanthin enriched *Haematococcus* biomass. As described above, the present disclosure aims to follow a strategically modified-form of life cycle of the organism by triggering the transformation of vegetative flagellated cells to pre-encysted intermediate palmelloid cells within 24 to 36 hours in defined nutrient sufficient conditions at any point of time of its life cycle, which can be either partly reverse back to introducing in to the inoculum development process that undergo multiple germination generating vegetative flagellates for faster inoculum development, and/or undergo simultaneous biomass growth and faster induction process of improved astaxanthin accumulation within 48 to 72 hours under the defined light spectrum simulation conditions.

**.** In a preferred embodiment, the present disclosure involves broad application of monochromatic red and far-red light based cultivation system design for enhancement of cell biomass and carotenoid (particularly astaxanthin) production, the method comprising:
a) inoculum development by cultivating *Haematococcus* cells in a growing solution/culture media containing full strength of freshwater algae culture media including but not limited to Bold basal medium with 3 fold nitrogen (3NBBM), Blue-Green 11 (BG11) medium, MES-Volvox medium, Tris-Acetate-Phosphate (TAP) medium, preferably modified form of original bold basal medium with 3 fold nitrogen and without vitamins (m3NBBM) under suitable conditions for optimal vegetative growth, wherein said conditions comprise growing the cells under far-red LED light and/or 1:1 mixture of cool white light and far-red LED light, preferably a mixture of 1:1 white light and far-red light, a light intensity in the range at least but not limited to between 80-200 *µ*mol photons.m⁻²s⁻¹, photoperiod of photoperiod of 12:12 or 14:10 or 16:8 or 18:6 or 24:0 hours (Light:Dark) cycle, preferably 18:6 hours, temperature in the range between 25 to 28°C, pH between 7.2 to 7.5, and continuous supply of air containing 2 to 5% CO₂; and
b) cultivating the cells grown according to step (a) under suitable conditions for optimal concurrent growth of biomass subsequently rapid induction and accumulations of astaxanthin in said cells, wherein said suitable conditions comprise exposing the cells grown in step (a) directly under continuous monochromatic light mixture of red and far-red at 1:1, 1:2, 2:1, 3:1 or 1:3 ratios, respectively, preferably at 2:1 ratio, or inoculating the cells grown in step (a) into a growing solution/culture media as stated in step (a) containing 1/3^{rd} of its full strength (with about 30 to 40 mgL⁻¹ N- content) under continuous monochromatic light mixture of red and far-red (at 1:1, 1:2, 2:1, 3:1 or 1:3 ratios, respectively, preferably 2:1 ratio) at a light spectrum of about 630 to 800 nm, a light intensity in the range at least but not limited to between 50-300 *µ*mol photons.m⁻²s⁻¹ , and essentially the all other above stated physical conditions under step (a).

. In an embodiment of the method of the present disclosure, a suitable growth reactor selected from a group comprising a vertical, a tubular or a flat panel photo-bioreactor is employed for rapid inoculum development and maintenance of vegetative flagellated cells as described above in step (a), and cultivation stage for concomitant biomass growth and astaxanthin accumulations as described above in step (b).

**.** In an exemplary embodiment of the present method, the cultivation of cells during inoculum development as described in step (a) above comprises growing the cells in a growing solution/culture media (m3NBBM) containing Na₂CO₃ (20 mgL⁻¹) and nitrogen (N) content (of about 120 mgL⁻¹), essentially a mixture of KNO₃ (30 mg L⁻¹) and NaNO₃ (90 mg L⁻¹), under conditions of white LED light supplemented with monochromatic far-red (at about 1:1 ratio) at a light spectrum/wavelength in the range between 340 nm to 850 nm and at a light intensity in the range between 80-200 *µ*mol photons.m⁻²s⁻¹, and a temperature of about 26 ± 2 °C.

. In another exemplary embodiment of the present method, the cultivation as described in step (b) of the cells grown above in step (a) comprises growth of cells in the range between 0.6 to 0.7 gL⁻¹ in a growing solution/culture media as stated above, essentially containing N-content in the range between 30 to 40 mgL⁻¹, under conditions of a mixture of monochromatic red and far red lights (at about 2:1 ratio) at a light spectrum/wavelength in the range between 630 to 800 nm and at light intensity in the range between 50 - 300 *µ*mol photons.m⁻²s⁻¹, wherein the temperature is maintained at about 26 ± 2 °C.

**.** In an embodiment, the results obtained in accordance with the present method indicate that the production rate of cells obtained by step (a) is in the range of about 0.5 to 0.6 gram/litre/day (gL⁻¹d⁻¹) of vegetative flagellate cells. Subsequently, exposure of the seed culture developed under step (a) to the aforementioned conditions of step (b) of the present method results in rapid biomass growth of over 0.6 to 0.7 gL⁻¹d⁻¹, wherein morphogenetic changes followed by induction of astaxanthin accumulations occur within 48 hours. The results further indicate that an average biomass production rate in the range between 0.5 to 0.7 gL⁻¹d⁻¹ can be maintained while simultaneous astaxanthin accumulations in the biomass was up to 5.8% and was in the average range between 3-6%, as determined from dry biomass. In an exemplary embodiment, the yield of astaxanthin was recorded up to 270 mgL⁻¹ and in the average range between 150 to 200 mgL⁻¹. The whole production cycle/method comprises of about 4 to 6 days, depending upon initial biomass density and desired biomass quality. The present inventors note that no cultivation process previously known has succeeded in providing concomitant enhancement of *Haematococcus* cell biomass enriched with such high astaxanthin content through one-stage/single phase cultivation, more particularly in about 4 to 6 days.

**.** In another embodiment, the method of the present disclosure comprises cultivation of *Haematococcus* cells according to steps (a) and (b), followed by chemical free harvesting or separating of the heavier palmelloids or astaxanthin containing *Haematococcus* cysts cultivated in step (b) by gravity settling, thereby promoting continuous harvesting and recycling of aquatic phase, thereby resulting in a substantial reduction in harvesting cost, reducing water footprint and harmful chemical free product development.

### . ADVANTAGES/BENEFITS:

The method for concomitant enhancement of cell biomass and carotenoid production in *Haematococcus* sp. as described in the present disclosure has several advantages/benefits, including, but not limiting to the following:
1. Control over life cycle stages: The complexity of normal life cycle stages of *Haematococcus* sp. (eg. *H. pluvialis*) is prevailing with morphogenetic transitions, which determines biomass growth stage by flagellate stage under optimal nutrient and growth conditions, followed by carotenoid (eg. astaxanthin) accumulating stage determined by suboptimal to nutrient deficient or stress conditions. Massive astaxanthin accumulation occurs at aplanospore stage post morphogenetic transitions from flagellates to intermediate palmelloids to aplanospores (Figure 1). The method of the present disclosure achieves modification of its normal life cycle stages with control over its normal morphogenetic changes of the life cycle at any point of time of its cultivation independent to its nutrient status. Herewith, subjective exposure of exponentially growing flagellates to specific combinations of light spectrum as described above expedites the process of required morphogenetic changes to palmelloids followed by astaxanthin induction. Relaxing the palmelloids to normal growth conditions reverses the process by inducing multiple divisions, hence allowing rapid increase of the population with newly germinated flagellates (Figure 2). Thus, the method of the present disclosure manages and controls the transition over flagellate, palmelloid and cysts (red) cell cycle stages, which is advantageous from a process point of view for faster inoculum development and ease of harvesting.
2. Concomitant enhancement of biomass and carotenoid (eg. astaxanthin) production: Quality biomass production with maximum yield is the most critical step in commercial production of high value products. A one-stage cultivation with concomitant enhancement of both biomass and astaxanthin is considered highly advantageous for commercial production due to its implications in simplicity in operating process compared to conventional two-stage cultivation method involving the necessity of primary harvest, nutrient removal stage and applications of cost intensive additional stress factors to induce astaxanthin. Considerable biomass loss during the stress applied induction stage and cross contaminations with other species owing to extensive cultivation durations are among the major constraints involving conventional two-stage cultivation. Thus, the current invention discloses a cultivation technique by applying strategic modulations of light spectrum qualities as described above, to achieve concomitant enhancement of biomass productivity and astaxanthin yield while improving the quality of the product with enhanced accumulation astaxanthin content, is a significant advantage (Figure 3). In particular, the present method is able to show a rapid one phase method of *Haematococcus* cultivation, including both biomass growth and accumulations of astaxanthin and other pigments, wherein the method produces results in only for about 6 to 7 days. In particular, biomass productivity improved up to 2-3 fold, with nearly 2 fold increase in astaxanthin content (up to 5.8%), and over a 3 fold increase in astaxanthin yield (up to 270 mgL⁻¹).
3. Rapid induction and astaxanthin accumulation: Cost of commercial astaxanthin production is directly related to its durations of production cycle. Minimizing the production cycle duration with improved astaxanthin content and yield is highly advantageous that positively impacts the overall operational cost reduction. The present method of rapid induction method while improving biomass and astaxanthin content concomitantly results in said advantage. Compared to 15 - 30 days in conventional methods using white light, the present method employing monochromatic far-red light, red light, or a mixture of monochromatic red and far-red lights, as described above result nearly about 3 to 4 times improvement in yield within about 6 to 7 days, while increasing the biomass astaxanthin content up to 5.8%. The minimum duration for astaxanthin induction was observed within 48 hours (Figure 4).
4. Reduction in the cost: The present disclosure describing concurrent cultivation method for biomass and astaxanthin production has positive implications and the cumulative economics of production is benefited significantly due to:
   i. Biomass growth and astaxanthin accumulations which is completed simultaneously in a single system under defined nutrient sufficient conditions, thus completely omitting the need of two separate systems for biomass growth and astaxanthin accumulation. Further, no primary or intermediate harvesting is required, which therefore reduces overall operation and handling costs significantly.
   ii. Rapid single-phase concurrent cultivation of biomass and astaxanthin accumulations shorten the total production cycle duration to about 6 to 7 days, thereby improving 2-3 fold increase in annual production as compared to conventional methods.
   iii. Improved biomass quality in single-stage cultivation step minimizes the additional costs of implementing a second step for improving quality of biomass products.
   iv. Significant reduction in nutrient inputs (eg. 1/3^{rd} of optimal growth medium) for complete production cycle without any additional encysting nutrient and vitamins result in significant reduction of production cost.
   v. Higher biomass growth, rapid induction and improved astaxanthin yield minimizes total cultivation durations from culture initiation to product development with significant reduction in cumulative cost of energy input.

**.** Thus, the present disclosure discloses a robust, controllable and simple technique to induce morphogenetic changes in green microalgae (particularly, *Haematococcus)* independent of the nutrient status compared to highly complex conventional methods that require a predefined nutrient management strategy or intermediate delimitation of nutrients. The present cultivation method is a standalone, rapid one-stage cultivation for high quality biomass production, either enriched with astaxanthin or with mixed carotenoids (lutein, β-carotene, canthaxanthin and astaxanthin). The present method of *Haematococcus* cultivation leads to high quality inoculum generation, improved biomass production, rapid induction and improved carotenoids (eg. astaxanthin) accumulations.

**.** Additional embodiments and features of the present disclosure will be apparent to one of ordinary skill in art based upon description provided herein. The embodiments herein provide various features and advantageous details thereof in the description. Descriptions of well-known/conventional methods and techniques are omitted so as to not unnecessarily obscure the embodiments herein. Further, the disclosure herein provides for examples illustrating the above described embodiments, and in order to illustrate the embodiments of the present disclosure certain aspects have been employed. The examples used herein for such illustration are intended merely to facilitate an understanding of ways in which the embodiments herein may be practiced and to further enable those of skill in the art to practice the embodiments herein. Accordingly, the following examples should not be construed as limiting the scope of the embodiments herein.

### EXAMPLES

. *Haematococcus pluvialis* strain (UTEX 2505) was employed in the present examples. Said *Haematococcus pluvialis* strain (UTEX 2505) was obtained/sourced from UTEX culture collection, Texas, USA.

### EXAMPLE 1:

### Concomitant biomass growth and carotenoid production

**.** Experiments were performed in Plant growth chamber (Percival LED Series) using Erlenmeyer flasks (250-500 ml) by cultivating *Haematococcus pluvialis* cells in a growing solution/culture media under suitable conditions for optimal vegetative growth of the cells, which served as the starting inoculum or seed culture for actual experiment in the second step below employing Red/Far-Red exposure. In particular, for development of starting inoculum or seed culture, full strength modified bold basal medium (BBM) was employed. The cells were exposed to a 1:1 mixture of cool white light and far-red LED light at a wavelength covering 340 nm to 850 nm, light intensity in the range between 80-200 *µ*mol photons.m⁻²s⁻¹, and a photoperiod of 12:12 or 14:10 or 16:8 or 18:6 or 24:0 hours (Light:Dark) cycle, preferably 18:6 hours (Light:Dark) cycle. The temperature of the process was maintained at 26 ±2 °C, pH in the range 7.2 to 7.5 with a continuous supply of air containing CO₂ in the range between 2 to 5%.

**.** The cells grown above were cultivated under suitable conditions for concurrent growth of biomass and rapid accumulation of astaxanthin in said cells. The process was carried out by: (i) exposing the above obtained cells (starting inoculum or seed culture) directly to a continuous monochromatic light mixture of red and far-red (preferably at 2:1 ratio) at a wavelength covering 630-800 nm (preferably 640 to 760 nm), *or* (ii) preferably by inoculating the cells grown above (starting inoculum or seed culture) into a growing solution/culture media containing 1/3^{rd} of the full strength of modified bold basal medium employed above (with about 30 to 40 mgL⁻¹ Nitrogen content). The cells were then exposed to continuous monochromatic light mixture of red and far-red (preferably at 2:1 ratio) at a wavelength covering 630-800 nm (preferably 640 to 760 nm), a light intensity in the range between but not limited to 50-300 *µ*mol photons.m⁻²s⁻¹, and including all other conditions as described above. Experiments were also conducted using white light alone under nutrient stress conditions (reflecting conventional approach), and a mixture of monochromatic red light and monochromatic far-red light under nutrient stress conditions to compare and analyze the impacts of induction methods on biomass and astaxanthin production. The results were compared with the effects of Red/Far-Red (R/FR) method as disclosed in the current invention, on concomitant biomass growth and astaxanthin yields.

**.** The results of the above experiments are depicted in Figures 3 and 4, respectively. As depicted in Figure 4, exposure to a mixture of red and far-red lights (R/FR) under nutrient sufficient conditions resulted in rapid induction of astaxanthin accumulation within 48-72 hours. In particular, improved astaxanthin accumulation of over 5% was observed under nutrient sufficient conditions when compared to the exposure to conventional white light alone, or a mixture of red light and far-red light under nutrient stress conditions. R/FR exposure under nutrient stress conditions produced the fastest astaxanthin induction but biomass productivity was less, resulting a lesser yield of astaxanthin compared to R/FR exposure under nutrient sufficient conditions. Hence, said results indicate that the present method disclosed herein requires an optimal nutrient sufficient/nutrient limited conditions for obtaining best results of simultaneous enhancement of biomass and astaxanthin yield.

. Additionally, results depicted in Figure 3 shows expedited astaxanthin production cycle through one-phase employment of a mixture of red and far red lights. In particular, concomitant enhancement of biomass growth and rapid astaxanthin production was observed in *Haematococcus* cells with supplementation of far red and red light under optimal nutrient sufficient/nutrient limited conditions. Biomass productivities and astaxanthin content enhanced rapidly under R/FR exposure with optimal nutrient sufficient/nutrient limited conditions. Moreover, astaxanthin production cycle was shortened up to 6 to 7 days with a yield of astaxanthin nearly about 3 to 4 times higher than conventional method employing white light exposure under nutrient deficient/stress conditions, or when the cells were exposed to mixture of red light and far-red light under nutrient deficient/stress conditions.

. The above results successfully showed the concomitant production of biomass along with carotenoids (astaxanthin) yields when the present method is employed.

. Additional embodiments and features of the present disclosure will be apparent to one of ordinary skill in art based on the description provided herein. The embodiments herein provide various features and advantageous details thereof in the description. Descriptions of well-known/conventional methods and techniques are omitted so as to not unnecessarily obscure the embodiments herein.

. The foregoing description of the specific embodiments fully reveals the general nature of the embodiments herein that others can, by applying current knowledge, readily modify and/or adapt for various applications such specific embodiments without departing from the generic concept, and, therefore, such adaptations and modifications should and are intended to be comprehended within the meaning and range of equivalents of the disclosed embodiments. It is to be understood that the phraseology or terminology employed herein is for the purpose of description and not of limitation. Therefore, while the embodiments in this disclosure have been described in terms of preferred embodiments, those skilled in the art will recognize that the embodiments herein can be practiced with modification within the spirit and scope of the embodiments as described herein.

. Throughout this specification, the word "comprise", or variations such as "comprises" or "comprising" wherever used, will be understood to imply the inclusion of a stated element, integer or step, or group of elements, integers or steps, but not the exclusion of any other element, integer or step, or group of elements, integers or steps.

. With respect to the use of substantially any plural and/or singular terms herein, those having skill in the art can translate from the plural to the singular and/or from the singular to the plural as is appropriate to the context and/or application. The various singular/plural permutations may be expressly set forth herein for sake of clarity.

. Any discussion of documents, acts, materials, devices, articles and the like that has been included in this specification is solely for the purpose of providing a context for the disclosure. It is not to be taken as an admission that any or all of these matters form a part of the prior art base or were common general knowledge in the field relevant to the disclosure as it existed anywhere before the priority date of this application.

. While considerable emphasis has been placed herein on the particular features of this disclosure, it will be appreciated that various modifications can be made, and that many changes can be made in the preferred embodiments without departing from the principles of the disclosure. These and other modifications in the nature of the disclosure or the preferred embodiments will be apparent to those skilled in the art from the disclosure herein, whereby it is to be distinctly understood that the foregoing descriptive matter is to be interpreted merely as illustrative of the disclosure and not as a limitation.

## Claims

1. A method for concomitant enhancement of cell biomass and carotenoid production in microalgal cells, said method comprising exposing the microalgal cells to monochromatic light selected from far-red light, red light, or a combination thereof.

2. The method according to claim 1, wherein the microalgae is *Haematococcus* sp., preferably *Haematococcus pluvialis;* and wherein the carotenoid selected from a group comprising astaxanthin, lutein, β-carotene, canthaxanthin, lycopene, violaxanthin, zeaxanthin and combinations thereof, preferably astaxanthin.

3. The method according to any of the preceding claims, wherein the *Haematococcus* cells are exposed to monochromatic far-red light at a wavelength ranging from about 700 nm to 800 nm, preferably about 730-760 nm.

4. The method according to any of the preceding claims, wherein the *Haematococcus* cells are exposed to monochromatic red light at a wavelength ranging from about 630 nm to 700 nm, preferably about 640-680 nm.

5. The method according to any of the preceding claims, wherein the *Haematococcus* cells are exposed to a combination of monochromatic far-red light and monochromatic red light at a wavelength ranging from about 630 nm to 800 nm, preferably about 640 nm to 760 nm.

6. The method according to any of the preceding claims, wherein the *Haematococcus* cells are exposed to monochromatic light selected from far-red light, red light or a combination thereof, for a time-period ranging from about 12 hours to 192 hours, preferably about from 48 hours to 144 hours.

7. The method according to any of the preceding claims, wherein said method for concomitant enhancement of cell biomass and carotenoid production in *Haematococcus* sp., comprises steps of:
a. exposing *Haematococcus* cells to white light, or monochromatic far-red light, or a combination of the white light and the monochromatic far-red light, to obtain starting seed cell biomass or inoculum;
b. exposing the cell biomass of step a) to monochromatic far-red light, or monochromatic red light, or a combination of the monochromatic far-red light and the monochromatic red light, for concomitant enhancement of cell biomass and carotenoid production.

8. The method according to claim 8, wherein the exposure to white light in step (a) is at a wavelength ranging from about 340 nm to 850 nm, and wherein the exposure to monochromatic far-red light in step (a) is at a wavelength ranging from about 700 nm to 800 nm, preferably about 730-760 nm.

9. The method according to claim 8, wherein the step (a) comprises inoculating the *Haematococcus* cells in a cell culture media and exposing under suitable conditions for a time-period ranging from about 48 hours to 72 hours to achieve optimal vegetative cell growth for starting seed cell biomass or inoculum production, wherein said suitable conditions comprise growing the cells under monochromatic far-red light at a wavelength between 700 nm to 800 nm or a 1:1 combination of cool white light and monochromatic far-red light at a wavelength between 340 nm to 850 nm, a light intensity in the range of about 80 to 200 *µ*mol photons.m⁻²s⁻¹, preferably in the range of about 100-160 *µ*mol photons.m⁻²s⁻¹; a photoperiod of about 12:12 or 14:10 or 16:8 or 18:6 or 24:0 hours (Light:Dark) cycle, preferably 18:6 hours, a temperature in the range of about 25°C to 28°C, a pH in the range of about 7.2 to 7.5, and a continuous supply of air containing about 2% to 5% CO₂.

10. The method according to claim 8, wherein the step (b) comprises: (i) exposing the *Haematococcus* cells grown according to the step (a) directly under continuous combination of monochromatic far-red light and monochromatic red light at 1:1, 1:2, 2: 1, 3:1 or 1:3 ratios, preferably at 2:1 ratio, and a wavelength between 630 nm to 800 nm, preferably 640 nm to 760 nm, for a time-period ranging from about 12 hours to 192 hours, preferably about 48 hours to 144 hours, a light intensity in the range of about 50 to 300 *µ*mol photons.m⁻²s⁻¹, a photoperiod of 12:12 or 14:10 or 16:8 or 18:6 or 24:0 hours (Light:Dark) cycle, preferably 24:0 hours, a temperature in the range of about 25°C to 28°C, a pH in the range of about 7.2 to 7.5, and a continuous supply of air containing about 2% to 5% CO₂, to achieve concomitant enhancement of cell biomass and astaxanthin production; or (ii) inoculating the *Haematococcus* cells grown according to the step (a) in a cell culture media, and exposing under suitable conditions for a time-period ranging from about 12 hours to 192 hours to achieve concomitant enhancement of cell biomass and carotenoid production, wherein said suitable conditions comprise growing the cells under the combination of monochromatic far-red light and monochromatic red light at 1:1, 1:2, 2: 1, 3:1 or 1:3 ratios, preferably at about 2:1 ratio, a wavelength between 630 nm to 800 nm, preferably 640 nm to 760 nm, a light intensity in the range of about 50 to 300 *µ*mol photons.m⁻²s⁻¹, a photoperiod of 12:12 or 14:10 or 16:8 or 18:6 or 24:0 hours (Light:Dark) cycle, preferably 24:0 hours, a temperature in the range of about 25°C to 28°C, a pH in the range of about 7.2 to 7.5, and a continuous supply of air containing about 2% to 5% CO₂.

11. The method according to claim 8, wherein the *Haematococcus* cells cultured in the step (a) are pre-encysted vegetative cells.

12. The method according to any of the preceding claims, wherein the method comprises concomitant enhancement of cell biomass and carotenoid production in *Haematococcus pluvialis,* comprising steps of:
a. exposing *Haematococcus pluvialis* cells to white light, or monochromatic far-red light, or a combination thereof, preferably the combination of white light and the monochromatic far-red light, to obtain starting seed cell biomass or inoculum; and
b. exposing the cell biomass of step a) to monochromatic far-red light, or monochromatic red light or a combination thereof, preferably the combination of monochromatic far-red light and the monochromatic red light, for concomitant enhancement of cell biomass and carotenoid production.

13. The method according to any of the preceding claims, wherein the method comprises concomitant enhancement of cell biomass and astaxanthin production in *Haematococcus pluvialis,* comprising steps of:
a. inoculating the *Haematococcus pluvialis* cells in a cell culture media and growing under suitable conditions for a time-period ranging from about 48 hours to 72 hours to achieve optimal vegetative cell growth to obtain starting seed cell biomass or inoculum, wherein said suitable conditions comprise growing the cells under 1:1 combination of cool white light and monochromatic far-red light at a wavelength between 340 nm to 850 nm, a light intensity in the range of about 80-200 *µ*mol photons.m⁻²s⁻¹, preferably about 100 to 160 *µ*mol photons.m⁻²s⁻¹, a photoperiod of 12:12 or 14:10 or 16:8 or 18:6 or 24:0 hours (Light:Dark) cycle, preferably 18:6 hours, a temperature in the range of about 25°C to 28°C, a pH in the range of about 7.2 to 7.5, and a continuous supply of air containing about 2% to 5% CO₂; and
b. inoculating the *Haematococcus pluvialis* cells grown according to the step (a) in a cell culture media, and growing under suitable conditions for a time-period ranging from about 12 hours to 192 hours, preferably about 48 hours to 144 hours to achieve concomitant enhancement of cell biomass and astaxanthin production, wherein said suitable conditions comprise growing the cells under continuous exposure of the combination of monochromatic far-red light and red light preferably at about 2:1 ratio, a wavelength between 630 nm to 800 nm, preferably between 640 nm to 760 nm, a light intensity in the range of about 50 to 300 *µ*mol photons.m⁻²s⁻¹, a photoperiod of about 12:12 or 14:10 or 16:8 or 18:6 or 24:0 hours (Light:Dark) cycle, preferably 24:0 hours, a temperature in the range of about 25°C to 28°C, a pH in the range of about 7.2 to 7.5, and a continuous supply of air containing about 2% to 5% CO₂.

14. The method according to any of the preceding claims, wherein the method comprises concomitant enhancement of cell biomass and astaxanthin production in *Haematococcus pluvialis,* comprising steps of:
a. inoculating the *Haematococcus pluvialis* cells in a cell culture media and growing under suitable conditions for a time-period ranging from about 48 hours to 72 hours to achieve optimal vegetative cell growth to obtain starting seed cell biomass or inoculum, wherein said suitable conditions comprise growing the cells under 1:1 combination of cool white light and monochromatic far-red light at a wavelength between 340 nm to 850 nm, a light intensity in the range of about 80-200 *µ*mol photons.m⁻²s⁻¹, preferably about 100 to 160 *µ*mol photons.m⁻²s⁻¹, a photoperiod of 12:12 or 14:10 or 16:8 or 18:6 or 24:0 hours (Light:Dark) cycle, preferably 18:6 hours, a temperature in the range of about 25°C to 28°C, a pH in the range of about 7.2 to 7.5, and a continuous supply of air containing about 2% to 5% CO₂; and
b. exposing the *Haematococcus* cells grown according to the step (a) directly under continuous combination of monochromatic far-red light and monochromatic red light at 1:1, 1:2, 2:1, 3:1 or 1:3 ratios, preferably at 2:1 ratio, and a wavelength between 630 nm to 800 nm, preferably 640 nm to 760 nm for a time-period ranging from about 12 hours to 192 hours, preferably about 48 hours to 144 hours, a light intensity in the range of about 50 to 300 *µ*mol photons.m⁻²s⁻¹, a photoperiod of 12:12 or 14:10 or 16:8 or 18:6 or 24:0 hours (Light:Dark) cycle, preferably 24:0 hours, a temperature in the range of about 25°C to 28°C, a pH in the range of about 7.2 to 7.5, and a continuous supply of air containing about 2% to 5% CO₂; to achieve concomitant enhancement of cell biomass and astaxanthin production.

15. The method according to any of the preceding claims, wherein said method is completed in a time-period ranging from about 2 days to 8 days, preferably in the range from 4 days to 6 days; wherein the method is performed in a cultivation apparatus or a photobioreactor (PBR) system selected from a group comprising Erlenmeyer flask/Conical flask, vertical photobioreactor, tubular photobioreactor, flat panel photobioreactor and combinations thereof; and wherein the average biomass growth rate ranges from about 0.5 to 0.7 gram/litre/day (gL⁻¹d⁻¹), and the astaxanthin yield ranges from about 150 to 270 mgL⁻¹.
